# EUROPEAN PATENT SPECIFICATION

(11) **EP 3 582 845 B1**
(45) Date of publication and mention of the grant of the patent: **06.01.2021**
(21) Application number: 18708526.1
(22) Date of filing: 17.02.2018
(51) Int. Cl.: A61M 25/09, A61B 5/00

(54) **MEDICAL DEVICE WITH PRESSURE SENSOR**
MEDIZINISCHE VORRICHTUNG MIT DRUCKSENSOR
DISPOSITIF MÉDICAL À CAPTEUR DE PRESSION

(30) Priority: 17.02.2017 US 201762460608 P
(43) Date of publication of application: 25.12.2019
(73) Proprietor: Boston Scientific Scimed, Inc., Maple Grove, MN 55311 (US)
(72) Inventor: VANCAMP, Daniel H., Elk River Minnesota 55330 (US); MEREGOTTE, Jose A., Blaine Minnesota 55449 (US); LARSEN, Steven R., Lino Lakes Minnesota 55110 (US); LEE, Gregory, Eden Prairie Minnesota 55346 (US); CORNWELL, Brian, Big Lake Minnesota 55309 (US)
(74) Representative: Vossius & Partner Patentanwälte Rechtsanwälte mbB
(86) International application number: PCT/US2018/018529
(87) International publication number: WO 2018/152429

(56) References cited:
- WO-A1-97/09926
- US-A1- 2007 255 145
- US-A1- 2013 274 618
- US-A1- 2014 276 109

## Description

### Technical Field

The present disclosure pertains to medical devices, and methods for manufacturing medical devices. More particularly, the present disclosure pertains to blood pressure sensing guidewires and methods for using pressure sensing guidewires.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

US 2007/255145 A1 relates to a sensor and guide wire assembly for intravascular measurements of a physiological variable in a living body, comprising: a sensor element and a sensor guide wire comprising a core wire and at least one signal transmitting cable connected to the sensor element, wherein a polymer layer is provided which encloses a portion of the core wire and said at least one signal transmitting cable.

US 2013/274618 relates to a guidewire system, comprising: a tubular guidewire having an open proximal end, a closed distal end, and a length extending therebetween, the tubular guidewire including at least one lumen extending from the proximal end to the distal end; and at least one pressure wire slidably disposed within the at least one lumen, the at least one pressure wire having a length and at least one pressure sensor disposed thereon; wherein the tubular guidewire includes a plurality of apertures disposed through an outer wall of the tubular guidewire.

US 2014/276109 A1 relates to a pressure sensing guidewire, comprising: a tubular member having a proximal portion and a distal portion; wherein the distal portion has a plurality of slots formed therein; wherein the distal portion has a first wall thickness along a first region and a second wall thickness different from the first wall thickness along a second region; and a pressure sensor disposed within the distal portion of the tubular member.

WO 97/09926 A1 relates to an intravascular device for measuring blood pressure and flow, comprising: an elongate shaft having a proximal end and a distal end, the distal end of the elongate shaft adapted to be inserted into the vasculature of a patient; a pressure transducer connected to the distal end of the elongate shaft; and a flow transducer connected to the distal end of the elongate shaft.

### Brief Summary

The present invention is defined by the claims.

This disclosure provides design, material, manufacturing method, and use alternatives for medical devices. An example medical device includes a pressure sensing guidewire. The pressure sensing guidewire comprises: a tubular member having a proximal region and a housing region; a pressure sensor disposed within the housing region; a signal transmitting member coupled to the pressure sensor and extending proximally therefrom; and a hydrophilic silicone coating disposed along an inner surface of the housing region.

Alternatively or additionally to any of the embodiments above, the pressure sensor includes an optical pressure sensor.

Alternatively or additionally to any of the embodiments above, the signal transmitting member includes an optical fiber.

Alternatively or additionally to any of the embodiments above, a sensor port is formed in the housing region that is positioned adjacent to the pressure sensor and wherein the hydrophilic silicone coating extends proximally of the sensor port.

Alternatively or additionally to any of the embodiments above, the proximal region of the tubular member is free of the hydrophilic silicone coating.

Alternatively or additionally to any of the embodiments above, further comprising a centering member coupled to the signal transmitting member.

Alternatively or additionally to any of the embodiments above, the hydrophilic silicone coating is designed so that blood contacting a surface of the hydrophilic silicone coating has a contact angle of about 50° or less.

Alternatively or additionally to any of the embodiments above, the hydrophilic silicone coating is designed so that blood contacting a surface of the hydrophilic silicone coating has a contact angle of about 30° or less.

Alternatively or additionally to any of the embodiments above, the hydrophilic silicone coating is designed so that blood contacting a surface of the hydrophilic silicone coating has a contact angle of about 10° or less.

Alternatively or additionally to any of the embodiments above, the tubular member has a first wall thickness along the housing region, wherein the tubular member has a second wall thickness along the proximal region, and wherein the first wall thickness is smaller from the second wall thickness.

Alternatively or additionally to any of the embodiments above, further comprising a centering member coupled to the signal transmitting member at a position adjacent to the pressure sensor.

Alternatively or additionally to any of the embodiments above, further comprising a tip member coupled to the housing region and extending distally therefrom.

A pressure sensing guidewire is disclosed. The pressure sensing guidewire comprises: a tubular member having a proximal region and a housing region; wherein the tubular member has a reduced wall thickness along the housing region; an optical pressure sensor disposed within the housing region; an optical fiber coupled to the optical pressure sensor and extending proximally therefrom; a hydrophilic coating disposed along an inner surface of the housing region; wherein the hydrophilic coating is designed so that blood contacting a surface of the hydrophilic coating has a contact angle of about 30° or less.

Alternatively or additionally to any of the embodiments above, a sensor port is formed in the housing region that is positioned adjacent to the optical pressure sensor and wherein the hydrophilic coating extends proximally of the sensor port.

Alternatively or additionally to any of the embodiments above, the proximal region of the tubular member is free of the hydrophilic coating.

A pressure sensing guidewire is disclosed. The pressure sensing guidewire comprises: a tubular member having a proximal region and a housing region; an optical pressure sensor disposed within the housing region; an optical fiber coupled to the optical pressure sensor and extending proximally therefrom; a hydrophilic silicone coating disposed along an inner surface of the housing region, the hydrophilic silicone coating extending from a distal end of the housing region to a position proximal of the optical pressure sensor; and wherein the hydrophilic silicone coating is designed so that a body fluid contacting a surface of the hydrophilic silicone coating has a contact angle of about 10° or less such that retention of air bubbles within the housing region is reduced.

Alternatively or additionally to any of the embodiments above, a sensor port is formed in the housing region that is positioned adjacent to the optical pressure sensor and wherein the hydrophilic silicone coating extends proximally of the sensor port.

Alternatively or additionally to any of the embodiments above, the proximal region of the tubular member is free of the hydrophilic silicone coating.

Alternatively or additionally to any of the embodiments above, the tubular member has a first wall thickness along the housing region, wherein the tubular member has a second wall thickness along the proximal region, and wherein the first wall thickness is smaller than the second wall thickness.

Alternatively or additionally to any of the embodiments above, further comprising a hydrophobic coating disposed along an outer surface of the tubular member.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
Figure 1 is a partial cross-sectional side view of a portion of an example medical device.
Figure 2 is a partial cross-sectional view of an example medical device disposed at a first position adjacent to an intravascular occlusion.
Figure 3 is a partial cross-sectional view of an example medical device disposed at a second position adjacent to an intravascular occlusion.
Figures 4-6 schematically illustrate a fluid interacting with a portion of an example medical device.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

During some medical interventions, it may be desirable to measure and/or monitor the blood pressure within a blood vessel. For example, some medical devices may include pressure sensors that allow a clinician to monitor blood pressure. Such devices may be useful in determining fractional flow reserve (FFR), which may be understood as the pressure after a stenosis relative to the pressure before the stenosis (and/or the aortic pressure).

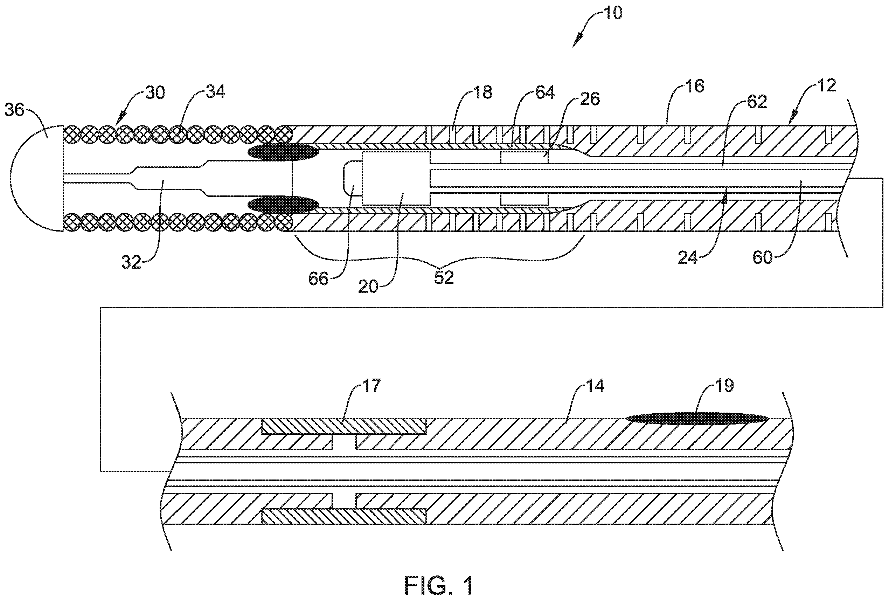

Figure 1 illustrates a portion of an example medical device 10. In this example, medical device 10 is a blood pressure sensing guidewire 10. However, this is not intended to be limiting as other medical devices are contemplated including, for example, catheters, shafts, leads, wires, or the like. Guidewire 10 may include a tubular member or shaft 12. Shaft 12 may include a proximal portion 14 and a distal portion 16. The materials for proximal portion 14 and distal portion 16 may vary and may include those materials disclosed herein. For example, distal portion 16 may include a nickel-cobalt-chromium-molybdenum alloy (e.g., MP35-N). Proximal portion 14 may be made from the same material as distal portion 16 or a different material such as stainless steel. These are just examples. Other materials are contemplated.

In some embodiments, proximal portion 14 and distal portion 16 are formed from the same monolith of material. In other words, proximal portion 14 and distal portion 16 are portions of the same tube defining shaft 12. In other embodiments, proximal portion 14 and distal portion 16 are separate tubular members that are joined together. For example, a section of the outer surface of portions 14/16 may be removed and a sleeve 17 may be disposed over the removed sections to join portions 14/16. Alternatively, sleeve 17 may be simply disposed over portions 14/16. Other bonds may also be used including welds, thermal bonds, adhesive bonds, or the like. If utilized, sleeve 17 used to join proximal portion 14 with distal portion 16 may include a material that desirably bonds with both proximal portion 14 and distal portion 16. For example, sleeve 17 may include a nickel-chromium-molybdenum alloy (e.g., INCONEL).

A plurality of slots 18 may be formed in shaft 12. In at least some embodiments, slots 18 are formed in distal portion 16. In at least some embodiments, proximal portion 14 lacks slots 18. However, proximal portion 14 may include slots 18. Slots 18 may be desirable for a number of reasons. For example, slots 18 may provide a desirable level of flexibility to shaft 12 (e.g., along distal portion 16) while also allowing suitable transmission of torque. Slots 18 may be arranged/distributed along distal portion 16 in a suitable manner. For example, slots 18 may be arranged as opposing pairs of slots 18 that are distributed along the length of distal portion 16. In some embodiments, adjacent pairs of slots 18 may have a substantially constant spacing relative to one another. Alternatively, the spacing between adjacent pairs may vary. For example, more distal regions of distal portion 16 may have a decreased spacing (and/or increased slot density), which may provide increased flexibility. In other embodiments, more distal regions of distal portion 16 may have an increased spacing (and/or decreased slot density). These are just examples. Other arrangements are contemplated.

A pressure sensor 20 may be disposed within shaft 12 (e.g., within a lumen of shaft 12). While pressure sensor 20 is shown schematically in Figure 1, it can be appreciated that the structural form and/or type of pressure sensor 20 may vary. For example, pressure sensor 20 may include a semiconductor (e.g., silicon wafer) pressure sensor, piezoelectric pressure sensor, a fiber optic or optical pressure sensor, a Fabry-Perot type pressure sensor, an ultrasound transducer and/or ultrasound pressure sensor, a magnetic pressure sensor, a solid-state pressure sensor, or the like, or any other suitable pressure sensor.

As indicated above, pressure sensor 20 may include an optical pressure sensor. In at least some of these embodiments, an optical fiber or fiber optic cable 24 (e.g., a multimode fiber optic) may be attached to pressure sensor 20 and may extend proximally therefrom. Optical fiber 24 may include a central core 60 and an outer cladding 62. In some instances, a sealing member (not shown) may attach optical fiber 24 to shaft 12. Such an attachment member may be circumferentially disposed about and attached to optical fiber 24 and may be secured to the inner surface of shaft 12 (e.g., distal portion 16). In addition, a centering member 26 may also be bonded to optical fiber 24. In at least some embodiments, centering member 26 is proximally spaced from pressure sensor 20. Other arrangements are contemplated. Centering member 26 may help reduce forces that may be exposed to pressure sensor 20 during navigation of guidewire and/or during use.

In at least some embodiments, distal portion 16 may include a region with a thinned wall and/or an increased inner diameter that defines a housing region 52. In general, housing region 52 is the region of distal portion 16 that ultimately "houses" pressure sensor 20. By virtue of having a portion of the inner wall of shaft 12 being removed at housing region 52, additional space may be created or otherwise defined that can accommodate sensor 20. Housing region 52 may include one or more openings 66 that provides fluid access to pressure sensor 20.

A tip member 30 may be coupled to distal portion 16. Tip member 30 may include a shaping member 32 and a spring or coil member 34. A distal tip 36 may be attached to shaping member 32 and/or spring 34. In at least some embodiments, distal tip 36 may take the form of a solder ball tip. Tip member 30 may be joined to distal portion 16 of shaft 12 with a bonding member 46 such as a weld.

Shaft 12 may include an outer coating 19. In some embodiments, coating 19 may extend along substantially the full length of shaft 12. In other embodiments, one or more discrete sections of shaft 12 may include coating 19. Coating 19 may be a hydrophobic coating, a hydrophilic coating, or the like.

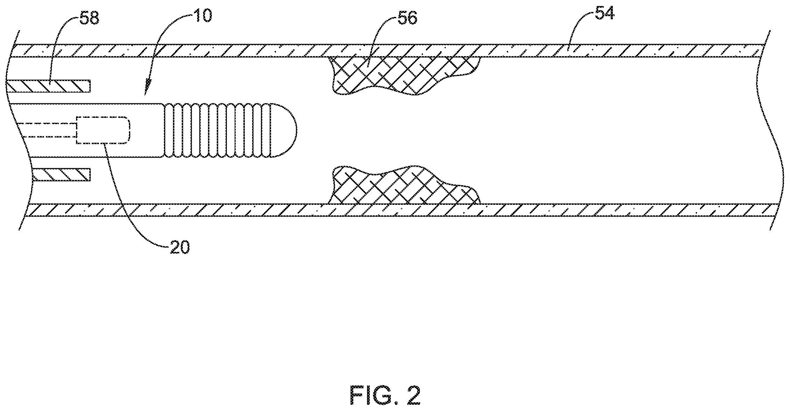

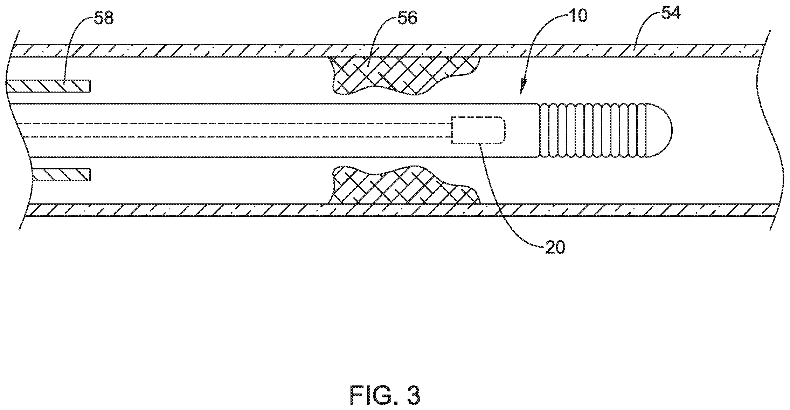

In use, a clinician may use guidewire 10 to measure and/or calculate FFR (e.g., the pressure after an intravascular occlusion relative to the pressure before the occlusion and/or the aortic pressure). Measuring and/or calculating FFR may include measuring the aortic pressure in a patient. This may include advancing guidewire 10 through a blood vessel or body lumen 54 to a position that is proximal or upstream of an occlusion 56 as shown in Figure 2. For example, guidewire 10 may be advanced through a guide catheter 58 to a position where at least a portion of sensor 20 is disposed distal of the distal end of guide catheter 58 and measuring the pressure within body lumen 54. This pressure may be characterized as an initial pressure. In some embodiments, the aortic pressure may also be measured by another device (e.g., a pressure sensing guidewire, catheter, or the like). The initial pressure may be equalized with the aortic pressure. For example, the initial pressure measured by guidewire 10 may be set to be the same as the measured aortic pressure. Guidewire 10 may be further advanced to a position distal or downstream of occlusion 56 as shown in Figure 3 and the pressure within body lumen 54 may be measured. This pressure may be characterized as the downstream or distal pressure. The distal pressure and the aortic pressure may be used to calculate FFR.

During the preparation for use and/or during the use of guidewire 10, it is possible that bubbles may be formed. For example, prior to inserting guidewire 10 into a patient, guidewire 10 may be flushed with a fluid (e.g., saline). During the flushing process, air bubbles may form. Some of these bubbles may become disposed within tubular member 12, for example within housing region 52. If the bubbles remain within tubular member 12, the bubbles could interact with pressure sensor 20 and possibly alter the pressure readings made by pressure sensor 20 and/or lead to the drifting of the pressure readings. It may be desirable to reduce or minimize these interactions and reduce pressure drift.

In order to reduce the amount of bubbles within tubular member 12, reduce drift, and/or otherwise improve the pressure readings made by pressure sensor 20, a hydrophilic coating 64 may be disposed along an inner surface of tubular member 12. Hydrophilic coating 64 may improve the wetting of the inner surface of tubular member and reduce the retention of bubbles. In at least some instances, hydrophilic coating 64 may be disposed adjacent to pressure sensor 20 and/or along housing region 52. For example, hydrophilic coating 64 may extend from the distal end of tubular member 12 to a position about 2-10cm (e.g., 5cm) proximally of pressure sensor 20. In some instances, hydrophilic coating 64 may extend proximally beyond housing region 52. This may include extending hydrophilic coating 64 down essentially the entire length of tubular member 12. Alternatively, proximal portion 14 of tubular member 12 may be free of hydrophilic coating 64. In some instances, hydrophilic coating 64 is disposed only along the inner surface of tubular member 12. In some of these and in other instances, hydrophilic coating 64 is disposed along other portions of tubular member 12 including along the outer surface, along both the inner surface and the outer surface, along pressure sensor 20, or along other suitable portions of tubular member 12. In some instances, the outer surface of tubular member 12 is free of hydrophilic coating 64. In some of these and in other instances, pressure sensor 20 is free of hydrophilic coating 64.

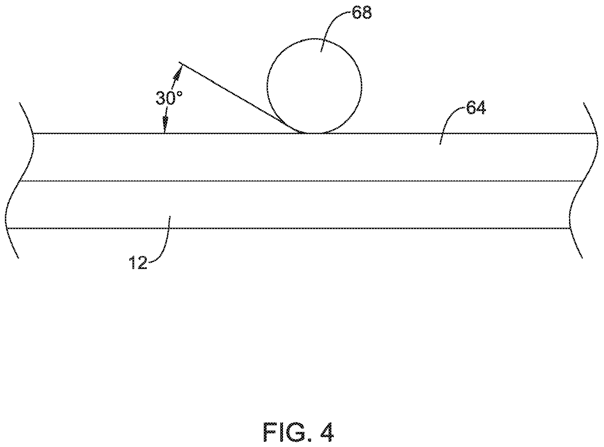

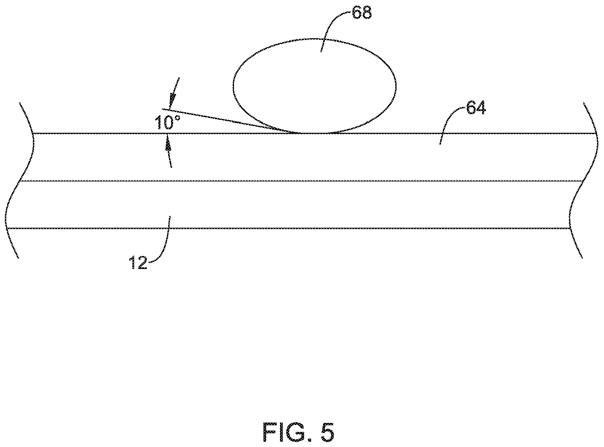

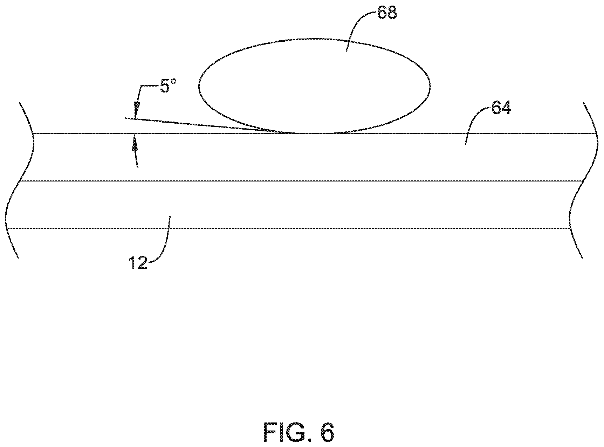

While not wishing to be bound by theory, hydrophilic coating 64 helps reduce bubble retention by reducing the contact angle between fluids contacting the surface of hydrophilic coating 64 and the surface of hydrophilic coating 64. In some instances, hydrophilic coating reduces the contact angle to less than about 90°, or about 50° or less, or to about 30° or less, or to about 15° or less, or to about 10° or less, or to about 5° or less, or to less than 10°, or to less than 5°. Figures 4-6 schematically illustrate the reduction in contact angle that can be achieved using coating 64. For example, Figure 4 illustrates a fluid droplet 68 in contact with hydrophilic coating 64. In this example, the contact angle may be about 30°. Figure 5 illustrates fluid droplet 68 contacting hydrophilic coating 64 with a contact angle of about 10°. Figure 6 illustrates fluid droplet 68 contacting hydrophilic coating 64 with a contact angle of about 5°.

A number of different materials are contemplated for hydrophilic coating 64. For example, in at least some embodiments, hydrophilic coating 64 may be a silicone-based hydrophilic material and, thus, form a hydrophilic silicone coating 64. One example silicone-based hydrophilic material is sold under the tradename SILPLEX (e.g., SILPLEX JQ-40), commercially available from Siltech LLC, Dacula, GA. In some of these and in other instances, hydrophilic coating 64 may be a hydrophobic material (e.g., a material that is typically seen as having hydrophobic properties) with hydrophilic properties. In some instances, the hydrophilic coating 64 may include a silicone polyether polymer with both hydrophilic and hydrophobic components (e.g., where the hydrophilic component may include polyethylene oxide, polypropylene oxide, combinations thereof, or the like; and where the hydrophobic component may include a siloxane backbone). In such instances, the ratio of polyethylene oxide and/or polypropylene oxide to siloxane can vary. Some examples of contemplated coating materials may include SILSURF A008, C208, B608, C410, D208, or the like. The surfactant concentrations may vary from about 0-8%. In some instances, the hydrophilic coating 64 may include a non-silicone material. For example, the hydrophilic coating may include a surfactant that may function as an anti-foam agent to destabilize air bubbles. In still other instances, the coating material 64 may include a polymethyl acrylate, polyvinylpyrrolidone, or the like. Other materials are contemplated.

Hydrophilic coating 64 may be disposed within tubular member 12 using a suitable method. For example, hydrophilic coating 64 may be applied to the inner surface of tubular member 12 using a dip coating process. For example, tubular member 12 may be dipped into a solution of hydrophilic coating 64 to a suitable depth (e.g. to a depth such that hydrophilic coating 64 extends proximally of pressure sensor 20). In some instances, the outer surface of tubular member 12 may be masked to avoid coating the outer surface. Alternatively, the outer surface of tubular member 12 may also be coated with hydrophilic coating 64. Upon completion, the integrity and coverage of hydrophilic coating 64 can be inspected and verified, for example, using a microscope. If desired, additional layers of hydrophilic coating 64 can be applied. Hydrophilic coating 64 can be dried at room temperature in air (e.g., within a carrier tube and/or product packaging). Alternatively, hydrophilic coating can be dried in an oven, in a modified atmosphere, combinations thereof, or the like.

The materials that can be used for the various components of guidewire 10 (and/or other guidewires disclosed herein) and the various tubular members disclosed herein may include those commonly associated with medical devices. For simplicity purposes, the following discussion makes reference to shaft 12 and other components of guidewire 10. However, this is not intended to limit the devices and methods described herein, as the discussion may be applied to other tubular members and/or components of tubular members or devices disclosed herein.

Shaft 12 and/or other components of guidewire 10 may be made from a metal, metal alloy, polymer (some examples of which are disclosed below), a metal-polymer composite, ceramics, combinations thereof, and the like, or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAM1D® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

In at least some embodiments, portions or all of guidewire 10 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of guidewire 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of guidewire 10 to achieve the same result.

In some embodiments, a degree of Magnetic Resonance Imaging (MRI) compatibility is imparted into guidewire 10. For example, guidewire 10, or portions thereof, may be made of a material that does not substantially distort the image and create substantial artifacts (e.g., gaps in the image). Certain ferromagnetic materials, for example, may not be suitable because they may create artifacts in an MRI image. Guidewire 10, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A pressure sensing guidewire (10), comprising:
a tubular member (12) having a proximal region (14) and a distal region (16), wherein a housing region (52) is included in the distal region (16);
a pressure sensor (20) disposed within the housing region;
a signal transmitting member coupled to the pressure sensor (20) and extending proximally therefrom; **characterized in that** the pressure sensing guidewire further comprises
a hydrophilic silicone coating (64) disposed along an inner surface of the housing region (52).

2. The pressure sensing guidewire of claim 1, wherein the pressure sensor includes (20) an optical pressure sensor.

3. The pressure sensing guidewire of any one of claims 1-2, wherein the signal transmitting member includes an optical fiber (24).

4. The pressure sensing guidewire of any one of claims 1-3, wherein a sensor port is formed in the housing region (52) that is positioned adjacent to the pressure sensor (20) and wherein the hydrophilic silicone coating (64) extends proximally of the sensor port.

5. The pressure sensing guidewire of any one of claims 1-4, wherein the proximal region (14) of the tubular member (12) is free of the hydrophilic silicone coating (64).

6. The pressure sensing guidewire of any one of claims 1-5, further comprising a centering member (26) coupled to the signal transmitting member.

7. The pressure sensing guidewire of any one of claims 1-6, wherein the hydrophilic silicone coating (64) is designed so that blood contacting a surface of the hydrophilic silicone coating (64) has a contact angle of about 50° or less.

8. The pressure sensing guidewire of any one of claims 1-6, wherein the hydrophilic silicone coating (64) is designed so that blood contacting a surface of the hydrophilic silicone coating (64) has a contact angle of about 30° or less.

9. The pressure sensing guidewire of any one of claims 1-6, wherein the hydrophilic silicone coating (64) is designed so that blood contacting a surface of the hydrophilic silicone coating (64) has a contact angle of about 10° or less.

10. The pressure sensing guidewire of any one of claims 1-9, wherein the tubular member (12) has a first wall thickness along the housing region (52), wherein the tubular member has a second wall thickness along the proximal region (14), and wherein the first wall thickness is smaller from the second wall thickness.

11. The pressure sensing guidewire of any one of claims 1-10, further comprising a centering member (26) coupled to the signal transmitting member at a position adjacent to the pressure sensor (20).

12. The pressure sensing guidewire of any one of claims 1-11, further comprising a tip member (30) coupled to the housing region (52) and extending distally therefrom.

13. The pressure sensing guidewire (10) of claim 1, wherein the tubular member has a reduced wall thickness along the housing region;
wherein the pressure sensor (20) includes an optical pressure sensor disposed within the housing region;
wherein the signal transmitting member includes an optical fiber (24) coupled to the optical pressure sensor (20) and extending proximally therefrom; and
wherein the hydrophilic coating (64) is designed so that blood contacting a surface of the hydrophilic coating (64) has a contact angle of about 30° or less.

14. The pressure sensing guidewire of claim 13, wherein a sensor port is formed in the housing region (52) that is positioned adjacent to the optical pressure sensor and wherein the hydrophilic coating (64) extends proximally of the sensor port.

15. The pressure sensing guidewire of any one of claims 13-14, wherein the proximal region (14) of the tubular member (12) is free of the hydrophilic coating (64).

## Patentansprüche

1. Druckerfassender Führungsdraht (10), der aufweist:
ein Röhrenteil (12) mit einem proximalen Bereich (14) und einem distalen Bereich (16), wobei ein Gehäusebereich (52) zum distalen Bereich (16) gehört;
einen Drucksensor (20), der im Gehäusebereich angeordnet ist;
ein signalübertragendes Teil, das mit dem Drucksensor (20) gekoppelt ist und sich davon proximal erstreckt;
**dadurch gekennzeichnet, dass** der druckerfassende Führungsdraht ferner eine hydrophile Silikonbeschichtung (64) aufweist, die entlang einer Innenfläche des Gehäusebereichs (52) angeordnet ist.

2. Druckerfassender Führungsdraht nach Anspruch 1, wobei der Drucksensor (20) einen optischen Drucksensor aufweist.

3. Druckerfassender Führungsdraht nach Anspruch 1 oder 2, wobei das signalübertragende Teil eine optische Faser (24) aufweist.

4. Druckerfassender Führungsdraht nach einem der Ansprüche 1 bis 3, wobei ein Sensorport im Gehäusebereich (52) gebildet ist, der benachbart zum Drucksensor (20) positioniert ist, und wobei sich die hydrophile Silikonbeschichtung (64) proximal vom Sensorport erstreckt.

5. Druckerfassender Führungsdraht nach einem der Ansprüche 1 bis 4, wobei der proximale Bereich (14) des Röhrenteils (12) frei von der hydrophilen Silikonbeschichtung (64) ist.

6. Druckerfassender Führungsdraht nach einem der Ansprüche 1 bis 5, ferner mit einem Zentrierteil (26), das mit dem signalübertragenden Teil gekoppelt ist.

7. Druckerfassender Führungsdraht nach einem der Ansprüche 1 bis 6, wobei die hydrophile Silikonbeschichtung (64) so gestaltet ist, dass Blut, das eine Oberfläche der hydrophilen Silikonbeschichtung (64) kontaktiert, einen Kontaktwinkel von etwa 50° oder weniger hat.

8. Druckerfassender Führungsdraht nach einem der Ansprüche 1 bis 6, wobei die hydrophile Silikonbeschichtung (64) so gestaltet ist, dass Blut, das eine Oberfläche der hydrophilen Silikonbeschichtung (64) kontaktiert, einen Kontaktwinkel von etwa 30° oder weniger hat.

9. Druckerfassender Führungsdraht nach einem der Ansprüche 1 bis 6, wobei die hydrophile Silikonbeschichtung (64) so gestaltet ist, dass Blut, das eine Oberfläche der hydrophilen Silikonbeschichtung (64) kontaktiert, einen Kontaktwinkel von etwa 10° oder weniger hat.

10. Druckerfassender Führungsdraht nach einem der Ansprüche 1 bis 9, wobei das Röhrenteil (12) eine erste Wanddicke entlang des Gehäusebereichs (52) hat, wobei das Röhrenteil eine zweite Wanddicke entlang des proximalen Bereichs (14) hat und wobei die erste Wanddicke kleiner als die zweite Wanddicke ist.

11. Druckerfassender Führungsdraht nach einem der Ansprüche 1 bis 10, ferner mit einem Zentrierteil (26), das mit dem signalübertragenden Teil an einer Position benachbart zum Drucksensor (20) gekoppelt ist.

12. Druckerfassender Führungsdraht nach einem der Ansprüche 1 bis 11, ferner mit einem Spitzenteil (30), das mit dem Gehäusebereich (52) gekoppelt ist und sich davon distal erstreckt.

13. Druckerfassender Führungsdraht (10) nach Anspruch 1, wobei das Röhrenteil eine reduzierte Wanddicke entlang des Gehäusebereichs hat;
wobei der Drucksensor (20) einen optischen Drucksensor aufweist, der im Gehäusebereich angeordnet ist;
wobei das signalübertragende Teil eine optische Faser (24) aufweist, die mit dem optischen Drucksensor (20) gekoppelt ist und sich proximal davon erstreckt; und wobei die hydrophile Beschichtung (64) so gestaltet ist, dass Blut, das eine Oberfläche der hydrophilen Beschichtung (64) kontaktiert, einen Kontaktwinkel von etwa 30° oder weniger hat.

14. Druckerfassender Führungsdraht nach Anspruch 13, wobei ein Sensorport im Gehäusebereich (52) gebildet ist, der benachbart zum optischen Drucksensor positioniert ist, und wobei sich die hydrophile Beschichtung (64) proximal vom Sensorport erstreckt.

15. Druckerfassender Führungsdraht nach Anspruch 13 oder 14, wobei der proximale Bereich (14) des Röhrenteils (12) frei von der hydrophilen Beschichtung (64) ist.

## Revendications

1. Fil-guide à détection de pression (10), comprenant :
un élément tubulaire (12) ayant une zone proximale (14) et une zone distale (16), dans lequel une zone de logement (52) est incluse dans la zone distale (16) ;
un capteur de pression (20) disposé dans la zone de logement ;
un élément de transmission de signal couplé au capteur de pression (20) et qui s'étend de manière proximale depuis celui-ci ; **caractérisé en ce que** le fil-guide à détection de pression comprend en outre
un revêtement en silicone hydrophile (64) disposé le long d'une surface intérieure de la zone de logement (52).

2. Fil-guide à détection de pression selon la revendication 1, dans lequel le capteur de pression comprend (20) un capteur de pression optique.

3. Fil-guide à détection de pression selon l'une quelconque des revendications 1 à 2, dans lequel l'élément de transmission de signal comprend une fibre optique (24).

4. Fil-guide à détection de pression selon l'une quelconque des revendications 1 à 3, dans lequel un port de capteur est formé dans la zone de logement (52) qui est positionnée de manière adjacente au capteur de pression (20), et dans lequel le revêtement en silicone hydrophile (64) s'étend de manière proximale par rapport au port de capteur.

5. Fil-guide à détection de pression selon l'une quelconque des revendications 1 à 4, dans lequel la zone proximale (14) de l'élément tubulaire (12) est exempte de revêtement en silicone hydrophile (64).

6. Fil-guide à détection de pression selon l'une quelconque des revendications 1 à 5, comprenant en outre un élément de centrage (26) couplé à l'élément de transmission de signal.

7. Fil-guide à détection de pression selon l'une quelconque des revendications 1 à 6, dans lequel le revêtement en silicone hydrophile (64) est conçu de sorte que le sang qui entre en contact avec une surface du revêtement en silicone hydrophile (64) possède un angle de contact d'environ 50° ou moins.

8. Fil-guide à détection de pression selon l'une quelconque des revendications 1 à 6, dans lequel le revêtement en silicone hydrophile (64) est conçu de sorte que le sang qui entre en contact avec une surface du revêtement en silicone hydrophile (64) possède un angle de contact d'environ 30° ou moins.

9. Fil-guide à détection de pression selon l'une quelconque des revendications 1 à 6, dans lequel le revêtement en silicone hydrophile (64) est conçu de sorte que le sang qui entre en contact avec une surface du revêtement en silicone hydrophile (64) possède un angle de contact d'environ 10° ou moins.

10. Fil-guide à détection de pression selon l'une quelconque des revendications 1 à 9, dans lequel l'élément tubulaire (12) possède une première épaisseur de paroi le long de la zone de logement (52), dans lequel l'élément tubulaire possède une seconde épaisseur de paroi le long de la zone proximale (14), et dans lequel la première épaisseur de paroi est inférieure à la seconde épaisseur de paroi.

11. Fil-guide à détection de pression selon l'une quelconque des revendications 1 à 10, comprenant en outre un élément de centrage (26) couplé à l'élément de transmission de signal à un emplacement adjacent au capteur de pression (20).

12. Fil-guide à détection de pression selon l'une quelconque des revendications 1 à 11, comprenant en outre un élément d'extrémité (30) couplé à la zone de logement (52) et s'étendant distalement depuis celle-ci.

13. Fil-guide à détection de pression (10) selon la revendication 1, dans lequel l'élément tubulaire possède une épaisseur de paroi réduite le long de la zone de logement ;
dans lequel le capteur de pression (20) comprend un capteur de pression optique disposé dans la zone de logement ;
dans lequel l'élément de transmission de signal comprend une fibre optique (24) couplée au capteur de pression optique (20) et s'étendant de manière proximale depuis celui-ci ; et
dans lequel le revêtement hydrophile (64) est conçu de sorte que le sang qui entre en contact avec une surface du revêtement hydrophile (64) possède un angle de contact d'environ 30° ou moins.

14. Fil-guide à détection de pression selon la revendication 13, dans lequel un port de capteur est formé dans la zone de logement (52) qui est positionnée de manière adjacente au capteur de pression optique, et dans lequel le revêtement hydrophile (64) s'étend de manière proximale par rapport au port de capteur.

15. Fil-guide à détection de pression selon l'une quelconque des revendications 13 à 14, dans lequel la zone proximale (14) de l'élément tubulaire (12) est exempte du revêtement hydrophile (64).
